# EUROPÄISCHE PATENTANMELDUNG

(11) **EP 2 020 220 A1**
(43) Veröffentlichungstag der Anmeldung: **04.02.2009**
(21) Anmeldenummer: 07113801.0
(22) Anmeldetag: 03.08.2007
(51) Int. Cl.: A61J 3/07

(54) **Dichtes Verschließen von gefüllten Arzneimittelkapseln**

(71) Anmelder: Boehringer Ingelheim Pharma GmbH & Co. KG, 55216 Ingelheim am Rhein (DE)
(72) Erfinder: Die Erfindernennung liegt noch nicht vor
(74) Vertreter: Hammann, Heinz

(57) **Zusammenfassung**

Die vorliegende Erfindung betrifft ein Verfahren, eine Vorrichtung und ein Steuerprogramm zum fluiddichten Verschließen von Kapseln enthaltend Arzneimittel, wobei die Kapseln aus wenigstens einem Kapselkörper und einer Kapselkappe bestehen, die teleskopartig ineinander gesetzt und im Stoßbereich auf der Kapselaußenseite mit einer dichtenden Banderole versehen werden, wobei die Kapselteile mit einem Gas befüllt werden, welches in Relation zur Umgebung eine geänderte Temperatur und/oder einen geänderten Druck aufweist und wobei ein Differenzdruckminderung in der Kapsel durch Spalte zwischen Kapselkörper und Kapselkappe nach dem ineinander Setzen der Kapselteile stattfindet.

Die durch das erfindungsgemäße Verfahren hergestellten Kapseln sind Einweg-Kapseln und enthalten bevorzugt eine Einzeldosis einer oral einnehmbaren pharmazeutischen Formulierung in Form eines Pulvers oder einer Flüssigkeit.

## Beschreibung

Die vorliegende Erfindung betrifft ein Verfahren, eine Vorrichtung und ein Steuerprogramm zum fluiddichten Verschließen von Kapseln enthaltend Arzneimittel, wobei die Kapseln aus wenigstens einem Kapselkörper und einer Kapselkappe bestehen, die teleskopartig ineinander gesetzt und im Stoßbereich auf der Kapselaußenseite mit einer dichtenden Banderole versehen werden.
Die durch das erfindungsgemäße Verfahren hergestellten Kapseln sind Einweg-Kapseln und enthalten bevorzugt eine Einzeldosis eines oral einnehmbaren Arzneimittels in Form eines Pulvers, einer Paste oder einer Flüssigkeit.
Kapseln mit pharmazeutischen Zubereitungen werden vielfältig in der Therapie und Diagnose von Krankheiten eingesetzt. Die Kapseln können oral verabreicht werden oder kommen in medizinischen Vorrichtungen zum Einsatz. In der Regel bestehen die Kapseln aus zwei Teilen, einem Kapselkörper (Körper) und einer Kapselkappe (Kappe), die teleskopartig ineinander geschoben werden. Aber auch mehrteilige Kapseln sind bekannt. Die Kapseln bestehen meistens aus Gelatine, insbesondere Hartgelatine. Für einige spezielle Anwendungen bestehen die Kapseln zuweilen auch aus für den Menschen gut verdaulichen, wasserlöslichen Kunststoffen, um bei oraler Verabreichung den Wirkstoff in bestimmten Kompartimenten des Magen-Darm-Trakts freizusetzen. Im folgenden sind Beispiele für verschiedene Kapselmaterialien aufgeführt.

EP 0460921 beschreibt Kapseln aus Chitosan und Stärke, Getreidepulver, Oligosacchariden, Methacrylsäure-Methylacrylat, Methacrylsäure-Ethylacrylat, Hydroxypropylmethyl-Celluloseacetat, -succinat oder -phthalat.
Das Kapselmaterial zeichnet sich dadurch aus, dass der Inhalt erst im Dickdarm freigesetzt wird.

GB 938828 offenbart Kapseln für radioaktive Substanzen im therapeutischen oder diagnostischen Einsatz. Die Kapseln bestehen aus wasserlöslicher Gelatine, Methylcellulose, Polyvinylalkohol oder wasserlöslichen nichttoxischen Thermoplasten.

Die EP0312760 beschreibt ein Verfahren zum Versiegeln von Hartgelatine oder Stärkekapseln mit einem bestimmten Siegelmittel. Die Naht der Kapseln kann dabei von der Mittelebene der Längsachse der Kapsel verschoben sein.

Die DE 3430764 offenbart ein anderes Verfahren zum Verschließen von Hartgelatinekapseln. Bei dem Verfahren werden die Kapseln zunächst gefüllt und die beiden Kapselhälften teleskopartig ineinander gesteckt.
Anschließend wird durch Anheben der Kapselkappe gegenüber dem Kapselkörper am Kapselkörper eine Kontaktzone freigelegt, wobei die Kapsel nicht geöffnet werden darf. In einem nächsten Schritt wird dann die Kontaktzone "klebrig" gemacht und anschließend wird die Kapselkappe wieder zurück auf ihre ursprüngliche Position geschoben und dabei mit der Kontaktzone in Berührung gebracht. Dieses Verfahren erfordert eine hohe Präzision bei der Ausführung, insbesondere da vermieden werden muss, die Kapsel beim Zurückführen der Kapselkappe auf den durch Erwärmung klebrig gemachten und damit gegenüber Verformung anfälligen Kapselkörper zu verformen. Auf Seite 32 der Anmeldung wird davon gesprochen, dass zum Halten und Führen der Kapselteile Werkzeuge notwendig sind, die keinerlei Toleranzen oder Spiel aufweisen.

Die zu befüllenden Kapseln werden auf Kapselabfüllmaschinen mit einem vorgegebenen Dosiervolumen möglichst homogen mit dem Medikament, das meist in flüssiger Form vorliegt, gefüllt. Dabei fließt die Dosiermenge in das Kapselunterteil, den Kapselkörper. Der Kapselkörper wird nach der Befüllung durch Aufstecken der Kapselkappe verschlossen.
Da die Kapselabfüllmaschinen mit hohen Taktraten arbeiten, geschieht das Aufstecken der Kapselkappe in wenigen Millisekunden. Problematisch dabei ist, dass das im unbefüllten Volumen des Kapselkörpers und insbesondere im unbefüllten Volumen der Kapselkappe befindliche Gas durch das ineinander Stecken komprimiert wird. Dieser Innendruck kann dazu führen, dass die Kapselteile wieder aufgeschoben werden.

Um dieses zu verhindern, können umlaufende ringförmige Vertiefungen im Kapselkörper und in der Kapselkappe angeordnet sein, die beim Aufstecken der Kappe auf den Körper ineinander greifen, wie die EP 1414639 B1 beschreibt. Zudem sind darin auch punktuelle Erhebungen und Vertiefungen beschrieben, die nach dem Aufstecken ineinander greifen und so einen festeren Halt ermöglichen.
Sollen Kapseln mit einem flüssigen Wirkstoff versehen werden, so müssen die Kapseln gegen ein Auslaufen gesichert sein. Hierzu ist eine Abdichtung der Kapseln nötig. Die Abdichtung kann beispielsweise durch ein Verschweißen der Kapselteile erfolgen, wie in der EP1414639 B1 dargestellt.

Weiterhin kann eine Abdichtung dadurch erfolgen, dass in oder auf den durch Kapselkörper und Kapselkappe gebildeten Spalt ein dichtender Kleber eingebracht wird oder eine Banderole außen auf die Kapsel im Bereich des Stoßes zwischen den Kapselteilen aufgetragen wird. Die Banderole besteht in der Regel aus dem gleichen Material wie die Kapselteile und wird durch Aufrollen oder Aufsprühen auf den Kapselmantel aufgetragen.
Steht das Gas im Kapselinneren unter Druck, so kann dies dazu führen, dass sich beim bzw. nach dem Auftragen der Banderolenflüssigkeit Fehlstellen in der aufgetragenen Banderole durch aus dem Innern der Kapseln am Stoß der Kappe austretendes Gas ergeben. Diese induzierte Gasströmung führt zur Ausbildung von Kanälen vom Kapselinneren nach Außen und wird beispielsweise anhand von Blasenbildung in der aufgetragenen Banderole am Stoß der banderolierten Kapsel beobachtet.
Wenn keine Kanäle entstehen, kann der vorhandene Überdruck dazu führen, dass sich die Kapsel infolge lokaler Überfeuchtung, gleichbedeutend mit einer Abnahme der Stabilität der Kapselwand am Ort des Banderolenauftrags, einseitig am Ort des Banderolenauftrags längt. Die in der Regel einseitige, d.h. ungleichmäßig über den Umfang der Kapsel, erfolgende Längung der Kapsel am Ort des Banderolenauftrags, resultiert schließlich in bananenförmig verbogenen Kapseln. Derart verborgene Kapseln sind nicht verpackbar und werden aussortiert.

Die CN1440740 beschreibt ein Verfahren zur Befüllung von Kapseln bestehend aus einer Kappe und einem Körper der mit einem flüssigen Präparat gefüllt wird, wobei die Abfüllung und das Aufbringen eines Siegelklebers unter erniedrigtem Druck erfolgen.

Die US 4,403,461 beschreibt ein Verfahren zum Versiegeln von Hartgelatinekapseln bestehend aus einem Kapselkörper und einer Kapselkappe, die miteinander verklebt werden. Zur Herstellung derartiger Kapseln sieht die US 4,403,461 vor, einen mit einer Membran überzogenen Stift zunächst in eine Dosierkammer für einen Kleber einzutauchen. Diese Kammer hat die Dimension eines Kapseloberteiles und weist am Wandinneren eine umlaufende mit Kleber gefüllte Rinne auf. Zur Aufnahme des Klebers wird die über den Stift gezogene Membran durch Kanäle im Stift aufgeblasen. Dabei wird der Kleber aus der Rinne auf die Membran aufgetragen. In einem zweiten Schritt wird der Stift mit Membran in eine Kapselkappe eingetaucht und die Membran auf den Innenumfang der Kapselkappe aufgeblasen. Dabei wird der ringförmige auf der Membran befindliche Kleberwulst auf die Innenfläche der Kapselkappe aufgebracht. In weiteren Schritten wird der Kapselkörper beispielsweise mit einem flüssigen Wirkstoff befüllt. Der befüllte Kapselkörper und die mit Kleber versehene Kapselkappe wird in eine evakuierbare Kammer transportiert und unter erniedrigtem Druck zusammengefügt.

Nachteilig an den insbesondere in der CN1440740 und US 4,403,461 beschriebenen Verfahren und Vorrichtungen ist der Effekt, dass mit ineinander Stecken der Kapselteile der beim Einstecken herrschende Druckzustand stationär sein muss, da durch den aufgebrachten Kleber sofort eine dichte Versiegelung erfolgt. Wird beispielsweise das Arzneimittel bei erhöhter Temperatur abgefüllt, was beispielsweise bei wachsartigen Pasten zur Dosierung nötig ist, so erhitzt sich das in der Kapsel befindliche Gas und es wird ein Druck aufgebaut, der auf den Dichtkleber wirkt. Um dies zu vermeiden, wären die Prozesszeiten alternativ so zu wählen, dass zunächst eine Abkühlung vor dem Verschließen der Kapsel erfolgen kann, was nachteilig lange Prozesszeiten bedingt. Weiterhin ist nachteilig, dass das Aufbringen des Klebers zur dichtenden Versiegelung nach der Lehre dieser Schriften schon vor dem Aufeinanderstecken erfolgt. Somit kann Kleber in das Kapselinnere gelangen und es ist erforderlich, den Schritt des Kleberauftrages in die Kapselbefüllung zu integrieren, was die Taktzeiten der Abfüllmaschine erniedrigt.

Daher besteht eine Aufgabe der Erfindung darin, ein Verfahren zum fluiddichten Verschließen von Arzneimittel enthaltenden Kapseln, bevorzugt flüssigen Arzneimitteln, zu schaffen, das einen dichten Verschluss der Kapseln bei einem im Vergleich zum Stand der Technik konstruktiv vereinfachten Prozess erlaubt.

Eine weitere Aufgabe besteht darin, eine Verschließverfahren zu schaffen, mit dem eine Leckage bei mit Arzneimitteln, vorzugsweise mit flüssigen Arzneimitteln, befüllten Kapseln vermieden wird.

Eine weitere Aufgabe besteht darin, eine Vorrichtung zur Durchführung des Verfahrens bereitzustellen, die es erlaubt, eine Banderole auf Kapseln, vorzugsweise flüssigkeitsgefüllte Kapseln, unter Vermeidung von Leckagen aufzubringen.

Eine weitere Aufgabe besteht darin, ein Verschließverfahren bereitzustellen, bei dem das Arzneimittel in der Kapsel durch das fluiddichte Verschließen nicht mit Klebestoffen kontaminiert wird und somit die pharmazeutische Qualität des Arzneimittels unberührt bleibt.

Die vorliegende Erfindung löst das oben beschriebene Problem, indem ein neues Verschließverfahren geschaffen wird, bei dem die Kapseln, nachdem diese mit dem Arzneimittel befüllt und zusammengefügt wurden, im Stoßbereich auf der Kapselaußenseite mit einer dichtenden Banderole versehen werden.
Kennzeichnend hierfür ist, dass die Kapselteile vor dem ineinander Stecken mit einem Gas, welches in Relation zur Umgebung eine geänderte Temperatur und/oder einen geänderten Druck aufweist, befüllt werden und auch nach dem ineinander Setzen der Kapselteile eine Differenzdruckminderung in der Kapsel durch Spalte zwischen Kapselkörper und Kapselkappe erfolgt.

Vorteilhaft im Vergleich zum Stand der Technik ist es hierdurch möglich, dass auch nach dem Verschließen noch ein Luftausgleich zur Umgebung erfolgen kann. Somit stellen sich deutlich niedrigere Anforderungen an ein erforderliches Vakuum in der Vorrichtung da auch nach dem ineinander Stecken der Kapselteile ein eventuell noch vorhandener Überdruck in der gesteckten Kapsel abgebaut werden kann.
Wenn ein erwärmtes Gas als Atmosphäre in den Kapseln mit eingeschlossen wird, dessen Druck sich im Folgenden durch Abkühlen des Gases verringert, so ist auch denkbar, dass Umgebungsgas von Außen in die Kapseln einströmt. Weiterhin erlauben das erfindungsgemäße Verfahren und die zugehörige Vorrichtung einen modularen Verschließprozess. Dabei können das Befüllen und das Zusammenfügen der Kapseln in der Kapselabfüllmaschine unter erniedrigtem Druck oder mit einem Prozessgas mit erhöhter Temperatur erfolgen. In einem zweiten Schritt findet dann das Aufbringen der Banderole statt.
Dies hat den Vorteil, dass im Vergleich zum Stand der Technik nur geringe konstruktive Änderungen an bekannten im Betrieb befindlichen Verschließvorrichtungen nötig sind.

Die nach dem Verfahren zu verschließenden Kapseln können aus synthetischen Polymeren, natürlicher und synthetischer Stärke oder a-1,4;a-1,6-glucan (Pullulan), sowie vorzugsweise aus Gelatine oder hydroxypropyl methycellulose (HPMC) bestehen, die die pharmazeutische Qualität der Inhaltsstoffe selbst nicht wesentlich beeinflussen, aber die Einsatzfähigkeit der gefüllten Kapsel im Hinblick auf ihre Funktion, die Dauer der Verwendung und/oder die Klimazone verbessern und in verschiedenen Stufen von der Herstellung bis zur Verwendung von Vorteil sind.

Gemäß der vorliegenden Erfindung fallen unter den Begriff Arzneimittel Arzneimittelwirkstoffe, Mischungen verschiedener Arzneimittel und Arzneimittelzusammensetzungen, sowie auch Arzneimittelformulierungen oder Kombinationen und Mischungen der vorgenannten Stoffe.

Die Kapsel besteht aus mindestens zwei Teilen, einem Kapselkörper (Körper) und mindestens einer Kapselkappe (Kappe), die so miteinander verbunden werden können, dass ein stabiler, abgeschlossener Hohlraum von definiertem Volumen gebildet wird, der die pharmazeutische Formulierung beinhaltet.

Bevorzugt besitzt das Material der Kapsel einen Permeationskoeffizienten für Wasserdampf von weniger als 10⁻¹³ kg/(m s Pa), bevorzugt weniger als 1,3 x 10⁻¹⁴ kg/(m s Pa). Bevorzugt liegt der Koeffizient zwischen 10⁻¹⁵ und 5 x 10⁻¹⁶ kg/(m s Pa), besonders bevorzugt zwischen 5 x 10⁻¹⁶ und 2 x 10⁻¹⁶ kg/(m s Pa). Der Vorteil dieser Eigenschaft besteht darin, dass somit verhindert wird, dass sich die Wasserkonzentration und somit die Arzneimittelkonzentration in der Kapsel ändert.

Kappe und Körper der Kapsel sind von gegenseitig kongruenter, zylinderartiger Form, bestehend aus einem in sich geschlossenen Mantel mit jeweils einer geschlossenen und einer offenen Seite. Dabei sind Form und Größe der Kappe und des Körpers dergestalt, dass der Körper mit seinem offenen Ende teleskopartig in das offene Ende der Kappe hinein geschoben werden kann.

In einer bevorzugten Ausführungsform sind in den Kapselkörper oder die Kapselkappe Beulen oder Dellen eingeprägt. Werden die mit derartigen Ausformungen und Einformungen versehenen Kapselteile ineinander gesteckt, so ergeben sich entlang der Berührungsfläche zwischen Kapselkörper und aufgesetzter Kapselkappe idealerweise definierte, einheitliche Spalten von 10 Mikrometer bis 500 Mikrometer, insbesondere 20 Mikrometer bis 50 Mikrometer. Die Spalten sind derart ausgeführt, dass einerseits gewährleistet ist, dass ein Gasausgleich und Druckausgleich durch Einströmen oder Ausströmen von Gas zwischen Umgebung und Kapselinnerem ermöglicht wird und andererseits kein Auslaufen der Befüllflüssigkeit erfolgt.

In speziellen Ausführungsformen sind Kappe und Körper mit Verschlusseinrichtungen versehen, die beim vorläufigen und/oder endgültigen Verschließen der Kapsel von Vorteil sind. In einer solchen Ausführungsform können sich auf dem Innenmantel der Kappe punktförmige Erhebungen und auf dem Außenmantel des Körpers etwas größere punktförmige Vertiefungen befinden, die so angeordnet sind, dass beim Verschließen der Kapsel die Erhebungen in die Vertiefungen einrasten. Alternativ können die Erhebungen auf dem Außenmantel des Körpers und die Vertiefungen auf dem Innenmantel der Kappe ausgebildet sein. Bevorzugt sind Anordnungen, bei denen die Erhebungen oder Vertiefungen jeweils ringförmig oder spiralförmig um den Mantel angeordnet sind. Anstelle der punktförmigen Gestaltung der Erhebungen und Vertiefungen können diese den Mantel der Kappe bzw. des Körpers ringförmig umlaufen, wobei aber vorteilhaft Aussparungen und Durchbrechungen vorgesehen sind, die einen Gasaustausch in und aus dem Kapselinneren ermöglichen.
In einer Ausführungsform sind auf dem Innenmantel der Kappe und dem Außenmantel des Körpers ein oder mehrere ringförmig umlaufende Erhebungen derart ausgebildet, dass sich im geschlossenen Zustand der Kapsel eine Erhebung der Kappe jeweils neben einer Erhebung des Körpers befindet.

In einer weiteren Ausführungsform sind auf der Außenseite des Körpers nahe dem offenen Ende Erhebungen und in der Kappe nahe dem offenen Ende Einformungen so ausgebildet, dass die Erhebungen des Körpers im geschlossenen Zustand der Kapsel in die Einformungen der Kappe einrasten. Die Erhebungen können dabei derart sein, dass die Kappe jederzeit ohne Beschädigung der Kapsel geöffnet werden kann oder aber dass die Kapsel nach einmaligem Verschließen nicht mehr zerstörungsfrei geöffnet werden kann.

Bevorzugt sind Kapseln mit einem oder mehreren solcher Einrastmechanismen (Rasten) (beispielsweise zwei umlaufende Rillen).

Besonders bevorzugt sind Kapseln mit wenigstens zwei solcher Einrastmöglichkeiten, die die beiden Kapselteile unterschiedlich stark fixieren. In einem solchen Teil kann eine erste Einrastmöglichkeit nahe den Öffnungen der Kapselkappe und des Kapselkörpers ausgebildet sein und eine zweite etwas weiter zum geschlossenen Ende der Kapselteile versetzt. Die erste Einrastmöglichkeit fixiert dabei die beiden Kapselteile weniger stark als die zweite.

Diese Variante hat den Vorteil, dass die Kapselkappe und der Kapselkörper nach dem Herstellen der Leerkapseln zunächst über den ersten Einrastmechanismus vorläufig miteinander verbunden werden können. Zum Befüllen der Kapsel werden dann die beiden Kapselteile wieder getrennt. Nach dem Befüllen werden die beiden Kapselteile soweit zusammengesteckt, bis der zweite Satz Rasten die Kapselteile fest fixiert.

In einer weiteren Ausführungsform ist auf der Außenseite des Körpers ein Wulst ausgebildet, der senkrecht zu der Verbindungsachse zwischen Kappe und Körper ringsum den Körper läuft. Der Wulst dient als Stopper für die Kappe, wenn diese über den Körper gesteckt wird, um ein Durchstoßen der Kappe mit dem Körper zu verhindern. Der Bereich zwischen offenem Ende des Körpers und dem Wulst entspricht dem Bereich des Körpers, über den die Kappe geschoben werden kann. Der Wulst ist so auf dem Körper lokalisiert, dass die Kappe weit genug über den Körper geschoben werden kann, um einen festen Verschluss zwischen Kappe und Körper zu bewirken. D.h. der Wulst befindet sich z.B. nicht unmittelbar an der offenen Seite des Körpers. Die Seite des Wulstes, die zum offenen Ende des Körpers zeigt, steht als senkrechte Kante so auf der Außenwand des Körpers, dass die Kappe beim Verschließen nicht über den Wulst hinweg geschoben werden kann. Die Seite des Wulstes, die zum geschlossenen Ende des Körpers weist, kann in Form einer nahezu rechtwinkeligen Kante ausgebildet sein oder sich zum geschlossenen Ende des Körpers hin verflachen. Die Ausbildung einer nahezu rechtwinkeligen Kante kann bei einer losen Einpassung der Kapsel in einen Kapselhalter von Vorteil sein, die Variante mit sich verflachendem Wulst bei einer festen Einpassung. Der Wulst ist für den Gasaustausch unterbrochen.

Die Stärke der Wände der Kappe und des Körpers können über den Gesamtbereich variieren. So ist die Wandstärke in der Regel in den abgerundeten Bereichen der Kappe oder des Körpers oder an der Stelle des Körpers, an der der Wulst ausgebildet ist, größer als in den Bereichen, in denen die Wände geradlinig verlaufen. In einer Ausführungsform haben die Wände der Kappe und des Körpers eine Dicke von 0,1 mm bis 0,5 mm, bevorzugt weist die Kapsel eine mittlere Wandstärke von 0,1 mm bis 0,4 mm, besonders bevorzugt 0,2 mm bis 0,4 mm auf.

Der Kapselkörper weist im Bereich seiner Öffnung, insbesondere an seiner Kante eine Dicke von 0,15 mm bis 0,35 mm, bevorzugt 0,225 mm bis 0,275 mm, besonders bevorzugt 0,25 mm auf.

Die Kapselkappe weist im Bereich ihrer Öffnung, insbesondere an ihrer Kante eine Dicke von 0,25 mm bis 0,45 mm, bevorzugt 0,325 mm bis 0,375 mm, besonders bevorzugt 0,35 mm auf.

Die Länge der Kapsel beträgt 8 mm bis 30 mm, bevorzugt 13 bis 17mm, besonders bevorzugt 15,5 mm bis 16 mm. Der Durchmesser der Kapsel beträgt 4 mm bis 7 mm, bevorzugt 5,3 mm bis 6,3 mm. Besonders bevorzugt 5,75 bis 5,95 mm.
Eine bevorzugte Kapsel weist eine Länge von 15,9 mm, einen Durchmesser des Kapselkörpers von 5,57 mm und einen Durchmesser der Kapselkappe von 5,83 mm auf. Die bevorzugte Wandstärke des Kapselkörpers beträgt 0,25 mm und die der Kapselkappe 0,35 mm.

Zum fluiddichten Verschließen wenigstens zweier teleskopartig ineinander einsetzbarer Teilelemente der Kapsel werden die zu befüllenden Kapselunterteile auf den Kapselabfüllmaschinen in Kapselträgern, insbesondere Matrizen gehalten. Dabei handelt es sich um zylindrische Formatteile aus Edelstahl, die unter anderem von Radialführungsstangen oder von einer Kette gehalten und bewegt werden. Das Kapselunterteil, der Kapselkörper sitzt in einer durchgehenden Bohrung. Ein Bund oder eine Verjüngung im Durchmesser der Bohrung verhindert, dass das Kapselunterteil nach unten durchrutscht. Zur Befüllung der Kapseln sind unterschiedliche Verfahren und Maschinen bekannt. Diese ähneln sich darin, dass sie vorwiegend volumetrisch, seltener gravimetrisch arbeiten. Ein vorgegebenes Dosiervolumen wird möglichst homogen mit dem Medikament, das beispielsweise als Flüssigkeit vorliegt, gefüllt. Meist wird dabei der Kapselkörper fast vollständig mit dem Wirkstoff gefüllt. Der Kapselkörper wird nach der Befüllung durch Aufstecken der Kappe verschlossen.
Die bekannten Kapselabfüllmaschinen arbeiten mit hohem Durchsatz, so dass bis zu 100000 Kapseln pro Stunde mit dem Arzneimittel befüllt werden.

Die zur Qualitätssicherung verwendeten Maßnahmen umfassen eine stichprobenweise Kontrolle der Kapseln in Hinblick auf die korrekte Füllmenge. Die Qualität der Kapseln wird auf der Grundlage der Stichproben und entsprechender statistischer Berechnung beurteilt. Meist erfolgt die Stichprobenmessung durch Wägung.
Wenn ein Überdruck in der Kapsel besteht, kann dieser dazu führen, dass die aufgesteckte Kapselkappe sich wieder löst und sich verschiebt. Um diese Verschiebung zu detektieren wird die Länge der Kapseln bestimmt. Weicht die gemessene Länge der Kapseln entlang der Längsachse durch die Kapsel mehr als 0,1 mm bis 1 mm, insbesondere 0,2 mm bis 0,4 mm von einer vorgegeben Solllänge ab, so wird die Kapsel aussortiert.

Um bei Kapseln mit flüssigen Arzneimittelfüllungen zu verhindern, dass die Flüssigkeit aus der Kapsel austritt, wird die Kapsel im Bereich des Stoßes der beiden Kapselteile versiegelt.

Hierbei können unterschiedliche Versieglungstechniken zum Einsatz kommen.
Beim Verschließen der Kapseln mit einer Banderole werden die mit dem Wirkstoff gefüllten, zusammengesteckten Kapseln in Fächern eines Transportbandes vereinzelt bewegt. Die Kapseln werden dabei liegend mit einer Banderolierband - Geschwindigkeit von 0,1 Meter pro Sekunde bis 2 Meter pro Sekunde, bevorzugt 0,4 Meter pro Sekunde bis 0,8 Meter pro Sekunde transportiert und drehen sich mit einer niedrigen Rotationsgeschwindigkeit um ihre eigene Längsachse. Diese Rotation wird hervorgerufen durch eine Bewegung der offenen Fächer im Transportband relativ zum unter den Fächern liegenden Boden. Durch eine Schrägstellung des Faches gegen die Laufrichtung wird erreicht, dass die Kapseln bei deren Rotation auch eine Kraftkomponente quer zur Laufrichtung erfahren, so dass die Kapseln gleichmäßig gegen eine Stirnseite der Fächer gedrückt werden. Hierdurch wird erreicht, dass sich der Stoßbereich der zusammengesteckten Kapseln an einer definierten Position befindet. An diesen Positionen sind die Fächer mit einer Ausnehmung versehen, so dass Banderolierscheiben, welche durch ein Bad mit einer Banderolenflüssigkeit laufen und dabei auf der umfangsseitigen Stirnseite Flüssigkeit aufnehmen, diese Flüssigkeit dann auf den Stoßbereich auftragen können. Das Auftragen geschieht dabei mittels zweier hintereinander geschalteter Banderolierscheiben. Im ersten Prozessschritt trägt eine erste Banderolierscheibe die Versiegelungslösung auf den Umfang der Kapsel auf. Die Flüssigkeit benetzt dabei die Kapselseite und kann aufgrund der Kapillarwirkung auch geringfügig in den Spalt eindringen. Vorteilhaft wird für zu banderolierende Hartgelatinekapseln eine Gelatinelösung mit einer Viskosität von 150cP bis 250 cP, insbesondere 180 cP bis 210 cP verwendet. Dabei wird die Lösung vorteilhaft bei einer Temperatur von 40 Grad Celsius bis 70 Grad Celsius, vorteilhaft 50 Grad Celsius bis 60 Grad Celsius aufgetragen. Um mögliche Fehlstellen dieser ersten Auftragung wie Blasen, fehlende Auftragslösung und Unebenheiten zu beseitigen und auszugleichen, wird von einer zweiten Banderolierscheibe ein nochmaliger Auftrag von Banderolierlösung durchgeführt. Eine weitere Möglichkeit des Versiegelns besteht durch Einbringen einer Siegellösung bevorzugt in den Spalt zwischen den Kapselteilen. Hierzu wird eine Siegellösung in die Fuge zwischen die zusammen gesteckten Kapselteile eingesprüht. Die Flüssigkeit ist hinsichtlich Ihrer Viskosität derart eingestellt, dass sie durch Kapillarkräfte in die Fuge einfließt und diese ringförmig vollständig ausfüllt. Überschüssige Flüssigkeit wird abgesaugt.

Zur Kontrolle der Dichtheit der Kapseln werden diese auf Fließtüchern in Ablagekästen einlagig geschichtet sowie nach Trocknung bzw. Verfestigung des Siegelbandes in einen Vakuumschrank verminderten Druckbedingungen ausgesetzt, um eine Leckage aus potentiell vorhandenen Fehlstellen im applizierten Band zu provozieren. Kapseln mit Leckagen zeichnen sich bei der Lagerung bzw. Testung unter reduzierten Druckbedingungen dadurch ab, dass das Fließ durchfeuchtet wird. Die Kapseln im Bereich der Leckage werden verworfen und die für dicht befundenen Kapseln verpackt.

Beim erfindungsgemäßen Verfahren geschieht die Kapselbefüllung und Kapselversiegelung bevorzugt modular, d.h. die Befüllung wird in einer Kapselabfüllvorrichtung vorgenommen und das Aufbringen der Banderole oder der Sprühversiegelung in einer Kapselversiegelungsvorrichtung.

Zur Befüllung werden einer Kapselabfüllmaschine leere Kapseln zugeführt, wobei das Kapseloberteil, die Kappe lose aufgesteckt ist. Die Kapseln werden von Kapselträgern aufgenommen und gehalten. Dann erfolgt eine Trennung der Kappe vom Körper und der Kapselkörper wird mit dem Arzneimittel gefüllt. Anschließend wird die Kapselkappe auf den gefüllten Körper aufgesetzt. Bedingt durch die hohe Prozessgeschwindigkeit erfolgt das Aufstecken der Kappe in wenigen Millisekunden. Hierbei wird das in dem Körper befindliche nicht befüllte Restgasvolumen und das Volumen des Gases in der Kappe auf das nicht befüllte Kapselinnenvolumen reduziert.

Um zu vermeiden, das hierbei ein unzulässiger Überdruck im Kapselinneren entsteht, wird erfindungsgemäß vorgeschlagen, das Aufstecken der Kappe unter einem geänderten Druck, insbesondere unter einem relativ zur Umgebung erniedrigten Druck vorzunehmen oder das die Kapselteile mit einem Gas erhöhter Temperatur zu befüllen, welches beim Erkalten an Druck verliert. Vorteilhaft sollte zusätzlich ein Spalt zwischen der Kappe verbleiben, der einen weiteren Druckdifferenzausgleich nach dem Aufstecken der Kappe erlaubt.

Bevorzugt wird in der Kapselabfüllmaschine ein erniedrigter Druck eingestellt. Hierzu kann vorgesehen sein, dass der Befüllraum oder Innenraum der Kapselabfüllmaschine mit einer Abdichtung zur Umgebung versehen wird. Dies können beispielsweise Klebebänder oder Kunststoffdichtungen, z.B. aus Silikon sein. Um einen definierten Unterdruck einstellen zu können, ist bevorzugt vorgesehen Druckmessgeräte in der Kapselabfüllmaschine und in der Umgebung anzuordnen. Die Druckdaten werden von einer Steuereinrichtung mit einem Datenspeicher gespeichert. Aus den Druckdaten wird in Abhängigkeit vom Kapselmaterial und vom abzufüllenden Arzneimittel und den Prozess- bzw. Maschinenparametern der Kapselabfüllmaschine ein Sollunterdruck errechnet und mittels einer geregelten Vakuumpumpe eingestellt. Als Vakuumpumpen sind Wasserstrahlabsaugung, Drehschieberpumpen, Rotationspumpen und Membranpumpen einsetzbar.
Alternativ ist es vorzugsweise auch vorgesehen, lokal einen Unterdruck zu erzeugen. Hierzu sind die Kapselhalter, Kapselmatrizen jeweils von einem druckdichten topfförmigen Gehäuse umgeben. Vor dem Zusammenstecken der Kapselteile werden die topfförmigen Gehäuse derart zusammengeführt, dass diese in eine zueinander dichte Lage gebracht werden. Konstruktiv kann dazu ein Gehäuseteil mit einem Rand und einer Dichtung versehen sein, die auf den Rand des Gegenparts aufliegt. Über Gas führende Leitungen wird dann ein definierter Unterdruck eingestellt, bzw. ein Prozessgas niedrigen Druckes eingeleitet.
Bevorzugt wird ein definierter Unterdruck in der Kapselabfüllmaschine von 50 Pa bis 5000 Pa, besonders bevorzugt von 100 Pa bis 500 Pa relativ zum Druck in der Umgebung eingestellt.
Um den Druck im Innenraum weitgehend konstant zu halten, ist vorteilhaft an oder in der Kapselabfüllmaschine eine Schleusenkammer angeordnet. Nachdem diese Schleusenkammer durch Pumpen auf denselben Unterdruck wie der Innenraum der Kapselabfüllmaschine gebracht wurde, werden die befüllten Kapseln in die Kammer eingeschleust, die Schleusenkammer wird dann mittels eines Verschlusses druckdicht zum Innenraum der Maschine verschlossen und dann zum Ausschleusen der Kapseln der Umgebungsdruck angelegt.
Alternativ oder in Ergänzung zum Anlegen von Unterdruck beim Zusammensetzen der Kapselteile ist vorzugsweise vorgesehen die Kapselteile vor dem Zusammensetzen mit einem erwärmten Gas zu befüllen.

Da sich das Gas nach dem Zusammenstecken der Kapselteile abkühlt, führt diese Abkühlung zu einer Druckminderung im in der Kapsel beim Aufstecken der Kapselkappe eingeschlossenen Gas und hilft so, Blasenbildung oder ein Aufbrechen der Versiegelung zu vermeiden. Das erwärmte Gas wird mit einer Düse eingestrahlt, deren Luftstrom in Richtung der offenen Kapselteile, insbesondere in Richtung der Kapselkappe gelenkt wird. Vorteilhaft wird als Prozessgas Stickstoff eingesetzt, welches auf eine Temperatur von 50 Grad Celsius bis 180 Grad Celsius, besonders bevorzugt 80 Grad Celsius bis 120 Grad Celsius erwärmt wurde.
Die Düse hat eine Breite von 5 cm bis 50 cm, besonders bevorzugt von 10 cm bis 30 cm. Nach dem Einsatz des erwärmten Gases soll die mit einem flüssigen Wirkstoff gefüllte Kapsel vor dem Versiegeln der Kapsel auf Umgebungstemperatur abgekühlt werden. Dazu ist vorgesehen, die Kapselhalter mit einer Kühlung zu versehen. Als Kühlung kann beispielsweise eine Wasserkühlung oder ein in den Kapselhalter integriertes Peltier-Element zum Einsatz kommen.

Um die Verfahrensschritte zur Kapselbefüllung und zur Versiegelung der Kapseln zu steuern weist die erfindungsgemäße Vorrichtung eine Steuereinrichtung, wie einen Mikrocontroller oder einen Steuercomputer auf. Zur Regelung des erfindungsgemäßen Verfahrens und der Steuereinrichtung entnimmt die Software die Prozessparameter zum Dosieren des Arzneimittels in die Kapselkörper einem Datenspeicher. Weiterhin erfasst die Software über an der Vorrichtung angeordnete Sensoren die Ist- Daten für Druck und Temperatur der Kapselfüllanlage und berechnet hieraus Soll-Daten für Druck und Temperatur des Prozessgases. Mittels Regeleinrichtungen, wie Vakuumpumpen oder Heizelementen und Kühlelementen regelt die Software über die Steuereinrichtung diese Soll-Daten ein.

Die unten genannten Verbindungen können allein oder in Kombination zur Anwendung in der erfindungsgemäßen Vorrichtung gelangen. In den unten genannten Verbindungen ist W einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von W kombiniert werden und zur Anwendung in der erfindungsgemäßen Vorrichtung gelangen. Beispielhaft genannte Kombinationen von W wären:
- W stellt ein Betamimetika dar, kombiniert mit einem Anticholinergika, Corticosteroide, PDE4-Inhibitore, EGFR-Hemmern oder LTD4-Antagonisten,
- W stellt ein Anticholinergika dar, kombiniert mit einem Betamimetika, Corticosteroiden, PDE4-Inhibitoren, EGFR-Hemmern oder LTD4-Antagonisten,
- W stellt ein Corticosteroiden dar, kombiniert mit einem PDE4-Inhibitoren, EGFR-Hemmern oder LTD4-Antagonisten
- W stellt ein PDE4-Inhibitoren dar, kombiniert mit einem EGFR-Hemmern oder LTD4-Antagonisten
- W stellt ein EGFR-Hemmern dar, kombiniert mit einem LTD4-Antagonisten.
   Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-14-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1 H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel AC-1 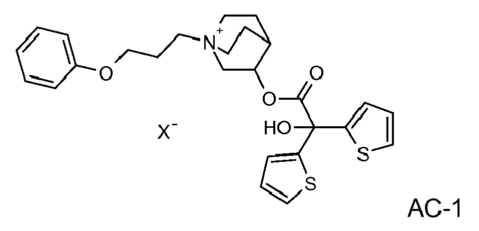 worin X - ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel AC-1-en 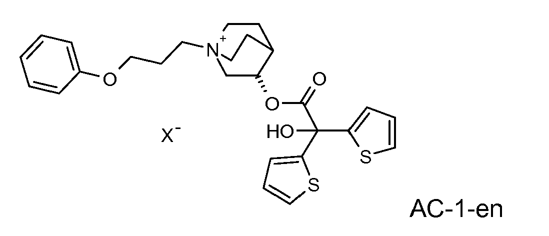 enthalten, worin X - die vorstehend genannten Bedeutungen aufweisen kann. Weiterhin bevorzugte Anticholinergika sind ausgewählt aus den Salzen der Formel AC-2 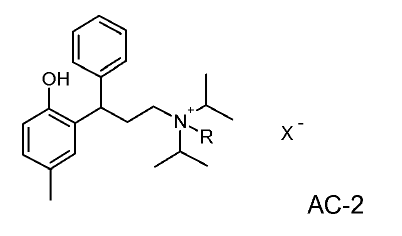 worin R entweder Methyl oder Ethyl bedeuten und worin X - die vorstehend genannte Bedeutungen aufweisen kann. In einer alternativen Ausführungsform kann die Verbindung der Formel AC-2 auch in Form der freien Base AC-2-base vorliegen. 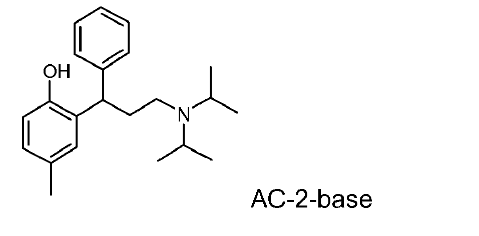 Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid
Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X- genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4aR*,10bS*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9H-pyrazolo[3,4-c]-1,2,4-triazolo[4,3-a]pyridin
- 9-Cyclopentyl-5,6-dihydro-7-ethyl-3-(tert-butyl)-9H-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]-amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinyl-carbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-ch inazol in
- 4-[(3-Chlor-4-fluor-phenyl)amino]-6-(trans-4-amino-cyclohexan-1-yloxy)-7-methoxy-ch inazol in
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yl-oxy}-7-methoxy-chinazolin
- 4-[(3-Chlor-4-fluor-phenyl)amino]-6-{1-[(methoxymethyl)carbonyl]-piperidin-4-yl-oxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-ch inazol in
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazol in
- 4-[(3-Chlor-4-fluor-phenyl)amino]-6-{trans-4-[(morpholin-4-yl)carbonylamino]-cyclohexan-1-yloxy}-7-methoxy-chinazol in
- 4-[(3-Chlor-4-fluor-phenyl)amino]-6-{trans-4-[(morpholin-4-yl)sulfonylamino]-cyclohexan-1-yloxy}-7-methoxy-chinazol in
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-ch inazol in
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-ch inazol in
- 4-[(3-Ethinyl-phenyl)amino]-6-[1-(tert.-butyloxycarbonyl)-piperidin-4-yloxy]-7-methoxy-ch inazol in
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazol in
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-ch inazol in
- 4-[(3-Chlor-4-fluor-phenyl)amino]-6-(cis-4-methylamino-cyclohexan-1-yloxy)-7-methoxy-ch inazol in
- 4-[(3-Chlor-4-fluor-phenyl)amino]-6-{cis-4-[N-(2-methoxy-acetyl)-N-methyl-amino]-cyclohexan-1-yloxy}-7-methoxy-chinazol in
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-ch inazol in
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethylamino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazol in
- 4-[(3-Chlor-4-fluor-phenyl)amino]-6-[cis-4-(N-acetyl-N-methyl-amino)-cyclohexan-1-yloxy]-7-methoxy-ch inazol in
- 4-[(3-Chlor-4-fluor-phenyl)amino]-6-(trans-4-methylamino-cyclohexan-1-yloxy)-7-methoxy-ch inazol in
- 4-[(3-Chlor-4-fluor-phenyl)amino]-6-[trans-4-(N-methansulfonyl-N-methyl-amino)-cyclohexan-1-yloxy]-7-methoxy-chinazol in
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-ch inazol in
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

### Beschreibung der Abbildungen

Die Abbildungen zeigen exemplarisch verschiedene Ausführungsformen von Kapseln für ein erfindungsgemäßes Verfahren und eine derartige Vorrichtung, dienen jedoch nur zur Illustration ohne den Umfang der Erfindung einzuschränken.
Fig. 1 zeigt eine einfache und bevorzugte Ausführungsform der beim erfindungsgemäßen Verfahren verwendeten Kapsel im lateralen Querschnitt.
Fig. 2a und 2b zeigen je eine unterschiedliche Ausführungsform der Kapsel mit sich verjüngendem Wulst am Körper im lateralen Querschnitt.
Fig. 3 zeigt eine Ausführungsform der Kapsel mit sich verjüngendem Wulst am Körper und punktförmigen Vertiefungen bzw. Erhebungen an Körper und Kappe in Frontalansicht.
Fig. 4 zeigt eine Ausführungsform der Kapsel mit sich verjüngendem Wulst am Körper und punktförmigen Erhebungen am Körper und punktförmigen Löchern in der Kappe in Frontalansicht.
Fig. 5 zeigt eine Ausführungsform einer Kapsel die durch Unterbrechungen in ringförmigen Vertiefungen einen Gasaustausch der Kapsel ermöglichen.
Fig.6 zeigt eine gefüllte Kapsel mit definiertem Spalt, die sich zur Versiegelung durch Aufsprühen eignet.
Fig. 7 und Fig. 8 zeigen das Auftragen einer Banderole auf eine gefüllte Kapsel in einem zweistufigen Prozess.
Fig. 9 zeigt eine Kapselbefülleinrichtung mit Vorrichtungen zum Erzeugen eines Vakuums und eines erwärmten Gasstromes.
Fig. 10 zeigt eine Steuervorrichtung zur Steuerung der Vorrichtungen und des Verfahrens.

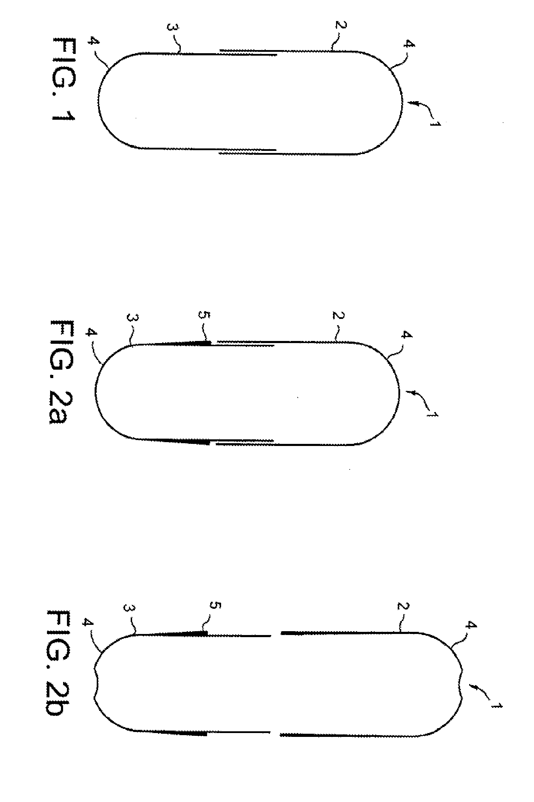

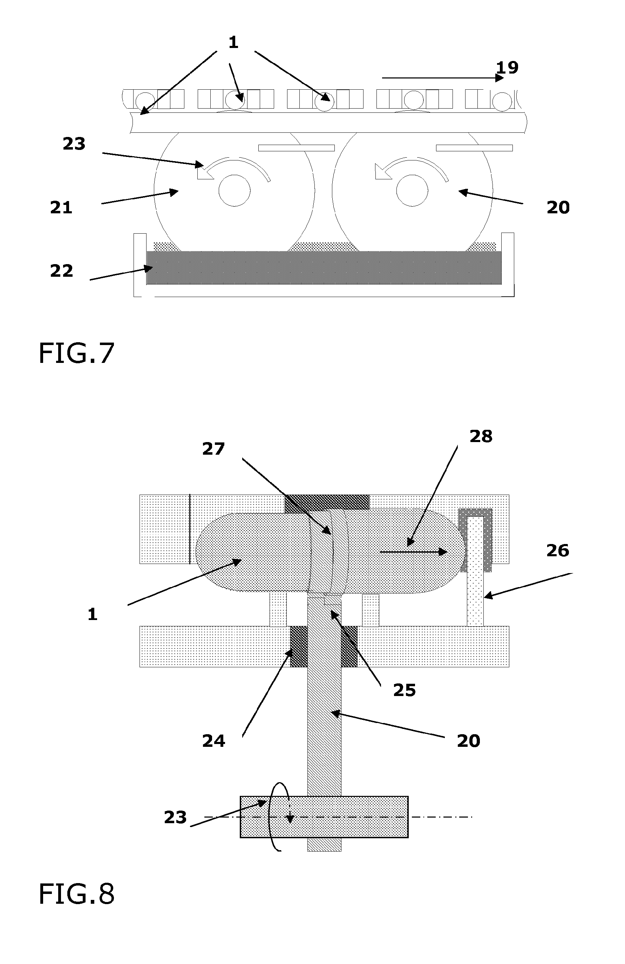

In Figur 1 ist eine einfache Ausführungsform der für das erfindungsgemäße Verfahren verwendeten Kapsel 1 im Querschnitt gezeigt. Die Kapsel 1 besteht aus der Kappe 2 und dem Körper 3, die teleskopartig ineinander gesteckt sind. Kappe 2 und Körper 3 sind von gleicher Gestalt und haben je eine konvexe Unterseite 4.
In Figur 2a ist im Querschnitt eine Ausführungsform gezeigt, bei der am Körper 3 der Kapsel 1 ein Wulst 5 ausgebildet ist, der sich zum geschlossenen Ende des Körpers hin verjüngt. Mit der zum offenen Ende des Körpers hin orientierten Seite steht der Wulst 5 nahezu senkrecht auf dem Körper. Die so ausgebildete Kante begrenzt den Bereich des Körpers über den die Kappe 2 teleskopartig geschoben werden kann. Eine andere Ausführungsform ist in Figur 2b abgebildet. Der Querschnitt zeigt, dass sich diese Ausführungsform von der in Figur 2a dargestellten dadurch unterscheidet, dass die Wandstärke der Kappe 2 bzw. des Körpers 3 nicht über den gesamten Bereich gleich stark ausgebildet ist, sondern über einzelne Teilbereiche variiert. Zusätzlich weisen die konvexen Unterseiten 4 der Kappe bzw. des Körpers je eine konkave Einbuchtung am Scheitelpunkt auf.

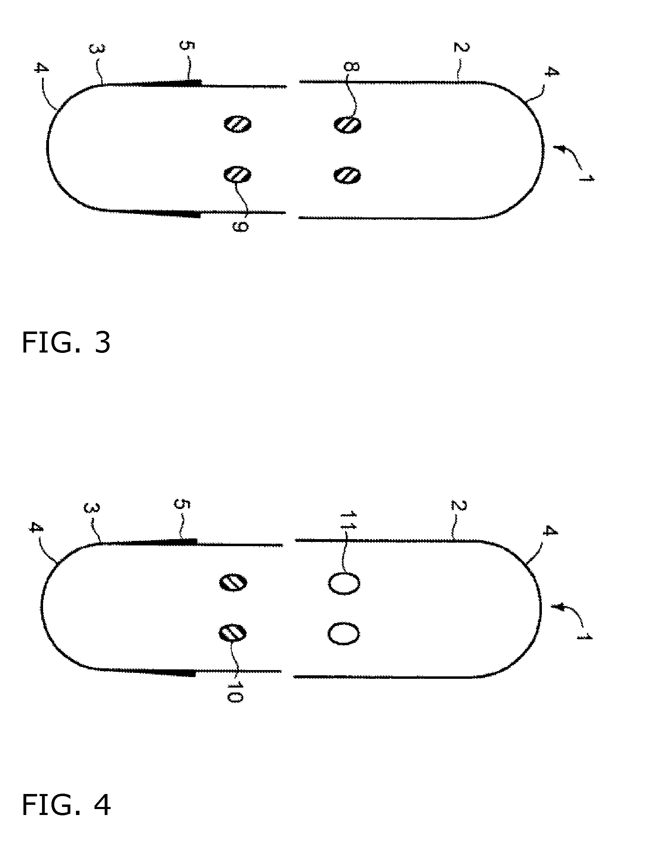

Figur 3 zeigt eine weitere Variante der Erfindung mit punktuellen Vertiefungen 8 und 9 als Frontalansicht.
In Figur 4 ist eine Variante der Kapsel 1 dargestellt, bei der am Körper 3 nahe dem offenen Ende Erhebungen 10 und in der Kappe 2 nahe dem offenen Ende Löcher 11 so ausgebildet sind, dass die Erhebungen 10 beim Verschließen der Kapsel in die Löcher 11 einrasten.

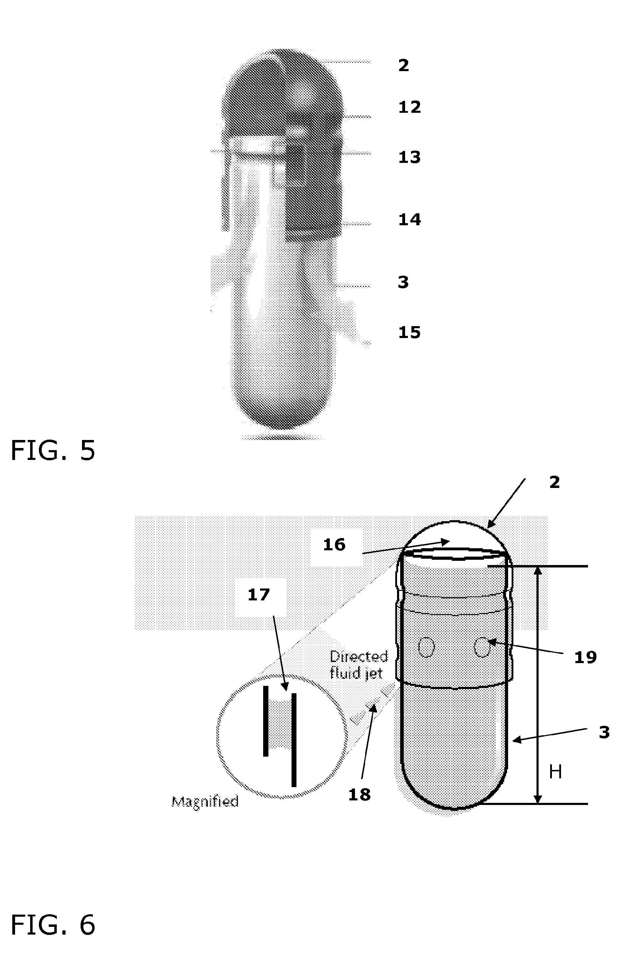

Figur 5 zeigt eine Kapsel mit einer Kappe 2 und einem Körper 3, wobei die Kappe eine obere ringförmige Vertiefung 12 aufweist, gegen die der Körper 3 anstößt. Weiterhin ist an der Kappe 2 ein Vorsteckring 13 in Form einer umlaufenden Vertiefung angeordnet, in die die untere ringförmige Vertiefung im vorgesteckten Zustand eingreift. Die umlaufenden Ringe 12, 13 und 14 sind nicht geschlossen umlaufend, sondern weisen ungeprägte Abschnitte auf, so dass dort ein gasdurchlässiger Spalt erhalten bleibt, welcher einen Druckdifferenzausgleich und einen Gasstrom 15 nach dem Aufstecken erlaubt.
Figur 6 zeigt eine Kapsel mit einer Kappe 2 und einem Körper 3 nach dem Befüllen und Zusammenstecken der Kapsel. Der Körper 3 der Kapsel ist bis zu einer Füllhöhe H befüllt worden. Insbesondere in der Kappe 2 wurde dabei ein Restgasvolumen 16 eingeschlossen. Über einen Entgasungsspalt 17 findet ein Gasaustausch zur Umgebung statt. Um einen definierten Abstand des Spaltes zu erzielen, sind Beulen 19 am Kapselkörper 3 oder Dellen 19 an der Kapselkappe 2 angeordnet. Die Wölbung der eingeprägten Beulen oder Dellen 19 zeigt in Richtung zum jeweils anderen Kapselteil. Auf den Stoßbereich wird eine Versiegelungsflüssigkeit in Form eines Flüssigkeitsstrahles 18 aufgesprüht. Durch Kapillarwirkung füllt die Versiegelungslösung den Spalt 17; überschüssige Versiegelungslösung wird abgesaugt.
Bei der Banderolierung und Versiegelung der Kapseln 1 werden diese mittels eines Gurtbandes entlang einer Laufstrecke 19 transportiert. In die Transportstrecke
greifen über Ausnehmungen 24 Banderolierscheiben 20, 21 ein, wie die Figuren 7 und 8 zeigen. Das Auftragen der Banderolierflüssigkeit aus einem Bad 22, in welches die Banderolierscheiben eintauchen, geschieht dabei zweistufig. Mit einer ersten Banderolierscheibe 20, die sich mit einer Rotationsrichtung 23 entgegen der Laufrichtung des Bandes dreht, wird ein erster Auftrag vorgenommen. Die Banderolierscheibe ist auf ihrer Radiusfläche entsprechend der Geometrie des Stoßes profiliert 25, um einen gleichmäßigen Auftrag der Versiegelung zu erreichen. Um eine definierte Lage des Kapselstoßes zu gewährleisten, erfährt die Kapsel 1 durch eine gegenüber der Laufrichtung geneigte Eigenrotation ein Moment 28 in Richtung einer Anschlagplatte 26, gegen die die Kappe gedrückt wird. Um eine höhere Sicherheit gegen Fehlstellen bei der Versiegelung zu erzielen erfolgt mit einer zweiten Banderolierscheibe 21 ein nochmaliger Auftrag von Banderolierflüssigkeit auf den Stoßbereich, so dass sich die endgültige Form der Banderole 27 ergibt, die nun getrocknet wird.

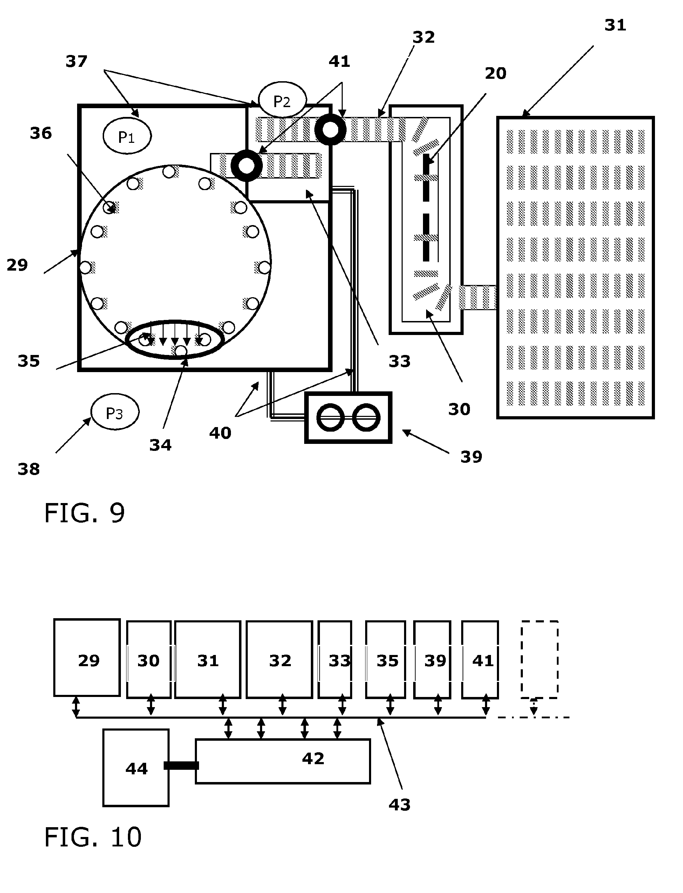

Figur 9 zeigt eine erfindungsgemäße Vorrichtung. In einer Kapselabfüllmaschine 29 erfolgt die Befüllung der Kapseln mit einem Medizinischen Wirkstoff. Hierzu werden die vorgesteckten Kapseln 1 von Kapselträgern 36 erfasst und gehalten, die Kapselkappe 2 vom Kapselkörper 3 abgezogen und der flüssige, halbfeste oder feste Wirkstoff in den Kapselkörper eingefüllt. Im nächsten Schritt werden die Kapselteile zusammengesteckt. Das Zusammenstecken geschieht in einem Gas mit zur Umgebung 38 um 300 Pascal erniedrigtem Druck P1. Um diese Druckdifferenz einzustellen, ist eine Vakuumpumpe 39 vorhanden, die über eine Prozessteuereinrichtung, wie einen Computer, oder eine SPS angesteuert wird.

Zur Erfassung des Druckes P1 in der abgedichteten Kapselabfüllmaschine sind Druckmesser 37 in der Füllmaschine angeordnet. Diese messen den Druckunterschied zur Umgebung Delta P = P2 - P1 und übermitteln diese Daten an die Steuereinrichtung. Um sicherzustellen, das in der Befüllvorrichtung 29 ein gleichmäßiger Druck herrscht, umfasst die Kapselabfüllmaschine eine gasdichte Schleuse 33 in die über Ventilklappen 41 die Kapseln ein und ausgeschleust werden. Vor dem Einschleusen von Kapseln aus der Füllmaschine wird die Vakuumschleuse 33 über eine Vakuumleitung 40 auf einen Druck P2 evakuiert, der dem Innendruck der Kapselabfüllmaschine entsprechen sollte. Dann wird die Schleuse mit Kapseln befüllt und zum Innenraum hin gasdicht verschlossen. Nun wird die Schleusenkammer nach Außen geöffnet und nimmt den Umgebungsdruck P3 an. Alternativ oder in Ergänzung zum Erzeugen eines Vakuums in der Kapselabfüllmaschine befindet sich eine Düse 34 im Bereich des ineinander Steckens der Kapselteile. Diese bläst einen auf Temperaturen von bis zu 110 Grad Celsius erwärmten Gasstrom 35 in die Kapselkappe ein. Die Kapsel wird dann im Kapselhalter 36 mittels eines in den Halter integrierten Kühlelementes (Peltierelement) auf 50 Grad Celsius abgekühlt.
Über Transfereinrichtungen 32 werden die Kapseln zu einer Versiegelungsmaschine transportiert. In einer Banderoliermaschine 30 werden die Kapseln durch Aufrollen einer Banderole versiegelt.
In einem Trockenschrank 31 wird die Versiegelung getrocknet. Geeignete Vorrichtungen und Verfahren zur Dichtheitsprüfung und zur Verpackung schließen sich an den Prozess an.
Mittels einer Steuereinrichtung 42, wie einem Prozessleitrechner oder einer SPS werden über ein Bussystem die Kapselabfüllmaschine 29, die Banderoliermaschine 30, die Trocknungsanlage 31, Transfereinrichtungen 32, eine Vakuumschleuse 33, Temperatur und Stärke eines Gasstroms 35, die Vakuumpumpe 39 und Vakuumventile 41 sowie weitere Prozessvorichtungen gesteuert, wie Figur 10 zeigt. Dabei wird die Steuereinrichtung mittels einer Software 44 geregelt, welche relevante Prozess- und Messdaten erfasst, verarbeitet und in einer Datenbank speichert, sowie die Vorrichtungen ansteuert.

### Beispiele

### Typische Betriebsdaten für ein Heißluftgebläse:

Heißluftgebläse 1800 W, elektronisch geregelt. Düse mit Düsenöffnung 30 mm x 250 mm, Heißlufttemperatur in Temperaturschritten von 2 Grad Celsius einstellbar zwischen 50 Grad Celsius und 180 Grad Celsius am Düsenaustritt.

### Typische Betriebsdaten für Vakuumpumpen:

Drehschieberpumpe Uno 200 von Pfeiffer mit 200 Kubikmeter Saugleistung pro Sunde oder Wälzkolbenpumpe WKP 250 von Pfeiffer mit 250 Kubikmeter Saugleistung pro Stunde oder Membranpumpe MVP 160 von Pfeiffer mit 10 Kubikmeter Saugleistung pro Stunde.

### Betriebsdaten für Kapselabfüllmaschinen und Versiegelungsmaschinen:

Die Betriebsdaten werden durch das jeweilige Fabrikat vorgegeben. Erreicht werden Füllleistungen von 100000 Kapseln pro Stunde.
Als Banderoliermaschine kann beispielsweise das Fabrikat Hicapseal 100 der Firma Qualicaps zum Einsatz kommen, die eine Kapazität von 80000 bis 100000 Kapseln pro Stunde besitzt.
Als Versiegelungsmaschine kann eine CFS 1200 der Firma Capsugel eingesetzt werden, bei welcher eine Versiegelung durch Aufsprühen der Versiegelung auf den Spalt zwischen den Kapselteilen erfolgt.

### Beispiele für Kapseln:

Länge der Kapselkörper: 22,2 ±0,46 mm; 20,22 ±0,46 mm; 20,98 ±0,46 mm; 18,4 ±0,46 mm; 16,61 ±0,46 mm; 15,27 ±0,46 mm; 13,59 ±0,46 mm; 12,19 ±0,46 mm; 9,3 ±0,46 mm.

Länge der Kapselkappe: 12,95 ±0,46 mm; 11,74 ±0,46 mm; 11,99 ±0,46 mm; 10,72 ±0,46 mm; 9,78 ±0,46 mm; 8,94 ±0,46 mm; 8,08 ±0,46 mm; 7,21 ±0,46 mm; 6,2 ±0,46 mm.

Äußerer Durchmesser der Kapselkörper: 9,55 mm; 8,18 mm; 7,36 mm; 7,34 mm; 6,63 mm; 6,07 mm; 5,57 mm; 5,05 mm; 4,68 mm.

Äußerer Durchmesser der Kapselkappen: 9,91 mm; 8,53 mm; 7,66 mm; 7,64 mm; 6,91 mm; 6,35 mm; 5,83 mm; 5,32 mm; 4,91 mm.

Gesamtlänge der geschlossenen Kapsel: 26,1 ±0,3 mm; 23,3 ±0,3 mm; 24,2 ±0,3 mm; 21,7 ±0,3 mm; 19,4 ±0,3 mm; 18,0 ±0,3 mm; 15,9 ±0,3 mm; 14,3±0,3 mm; 11,1 ±0,3 mm.

Kapselvolumina: 1,37 ml; 1,02 ml; 0,95 ml; 0,91 ml; 0,78 ml; 0,61 ml; 0,59 ml; 0,50 ml; 0,43 ml; 0,37 ml; 0,33 ml; 0,30 ml; 0,26 ml; 0,21 ml; 0,18 ml; 0,13 ml.

Gewicht der Kapseln: 163 mg; 118 mg; 110 mg; 96 mg; 76 mg; 61 mg; 48 mg; 38 mg; 28 mg.

## Patentansprüche

1. Verfahren zum fluiddichten Verschließen von Kapseln enthaltend Arzneimittel, wobei die Kapseln aus wenigstens einem Kapselkörper und einer Kapselkappe bestehen, die teleskopartig ineinander gesetzt und im Spalt des Stoßes auf der Kapselinnenseite mit einer dichtenden Versiegelung oder auf der Kapselaußenseite mit einer dichtenden Banderole versehen werden, **gekennzeichnet durch** die Verfahrensschritte
• Befüllen der Kapselteile mit einem Gas, welches in Relation zur Umgebung eine geänderte Temperatur und/oder einen geänderten Druck aufweist
• Differenzdruckminderung in der Kapsel **durch** Spalte zwischen Kapselkörper und Kapselkappe nach dem ineinander Setzen der Kapselteile.

2. Verfahren nach Anspruch 1, **dadurch gekennzeichnet, dass** die Kapselteile mit einem Gas mit einem relativen Unterdruck von 50 Pa bis 5000 Pa, vorzugsweise 100 Pa bis 500 Pa befüllt werden.

3. Verfahren nach einem der Ansprüche 1 oder 2, **dadurch gekennzeichnet, dass** der Innenraum einer Kapselabfüllmaschine mittels einer Pumpe, insbesondere Vakuum- Drehschieberpumpe evakuiert wird.

4. Verfahren nach einem der vorhergehenden Ansprüche, **dadurch gekennzeichnet, dass** die Kapseln über eine Schleusenkammer aus der Kapselabfüllmaschine transportiert werden, wobei die Schleusenkammer beim Einschleusen der Kapseln aus der Kapselabfüllmaschine auf einen zum Innern der Maschine gleichen Unterdruck gebracht, die Schleusenkammer dann druckdicht zum Innern verschlossen, sowie zum Ausschleusen auf Umgebungsdruck gebracht wird.

5. Verfahren nach Anspruch 1, **dadurch gekennzeichnet, dass** die Kapselteile mit einem erwärmten Gas befüllt werden.

6. Verfahren nach Anspruch 5, **dadurch gekennzeichnet, dass** das Gas eine Temperatur von 50 Grad Celsius bis 180 Grad Celsius, insbesondere 80 Grad Celsius bis 120 Grad Celsius aufweist.

7. Verfahren nach einem der Ansprüche 5 oder 6, **dadurch gekennzeichnet, dass** das erwärmte Gas nach der Befüllung mit dem Arzneimittel mittels einer Düse in die offenen Kapselteile, insbesondere in Richtung der Kapselkappe eingestrahlt wird, bevor diese ineinander gesetzt werden.

8. Verfahren nach einem der Ansprüche 5, 6 oder 7, **dadurch gekennzeichnet, dass** die Kapseln vor dem Aufbringen der Banderole auf eine Temperatur von 20 Grad Celsius bis 60 Grad Celsius abgekühlt werden.

9. Verfahren nach einem der vorhergehenden Ansprüche, **dadurch gekennzeichnet, dass** in den Kapselkörper oder die Kapselkappe Beulen oder Dellen eingeprägt sind, derart, dass die Kapsel entlang der Berührungsfläche zwischen Kapselkörper und aufgesetzter Kapselkappe definierte Spalten von 10 Mikrometer bis 500 Mikrometer, insbesondere 20 Mikrometer bis 50 Mikrometer aufweist.

10. Verfahren nach einem der vorhergehenden Ansprüche, **dadurch gekennzeichnet, dass** das Kapselmaterial aus Gelatine oder hydroxypropyl methylcellulose (HPMC) besteht.

11. Verfahren nach einem der vorhergehenden Ansprüche, **dadurch gekennzeichnet, dass** die die Kapselwandung eine Dicke von 0,05 mm bis 0,5 mm bevorzugt 0,1 mm bis 0,4 mm, besonders bevorzugt von 0,2 mm bis 0,4 mm aufweist.

12. Verfahren nach einem der vorhergehenden Ansprüche, **dadurch gekennzeichnet, dass** die Kapsel eine Länge von 8 mm bis 30 mm, bevorzugt 13 bis 17 mm und besonders bevorzugt von 15,5 mm bis 16 mm aufweist und einen Durchmesser 4 mm bis 7 mm, bevorzugt 5,3 mm bis 6,3 mm aufweist.

13. Verfahren nach einem der vorhergehenden Ansprüche, **dadurch gekennzeichnet, dass** eine Banderole zweistufig durch Banderolierscheiben aufgetragen wird.

14. Verfahren nach einem der vorhergehenden Ansprüche 1 bis 12, **dadurch gekennzeichnet, dass** eine Versiegelung in den Spalt zwischen Kapselkappe und Kapselkörper aufgesprüht und mittels Kapillarwirkung eingebracht wird.

15. Verfahren nach einem der vorhergehenden Ansprüche, **dadurch gekennzeichnet, dass** als dichtende Banderole eine wässrige Gelatinelösung oder eine HPMC Lösung auf Äthanolbasis aufgetragen wird.

16. Vorrichtung zum Durchführen des Verfahrens nach einem der Ansprüche 1 bis 15 umfassend mindestens eine Kapselabfüllmaschine und eine Maschine zum Aufbringen einer Banderole, **dadurch gekennzeichnet, dass** die Vorrichtung weiterhin Mittel zum Einstellen eines definierten Unterdruckes von 50 Pa bis 5000 Pa, vorzugsweise 100 Pa bis 500 Pa relativ zur Umgebung und/oder Mittel zum Befüllen der Kapselteile mit einem erwärmten Gas aufweist.

17. Vorrichtung nach Anspruch 16, **dadurch gekennzeichnet, dass** der Innenraum der Kapselabfüllmaschine eine Abdichtung zur Umgebung aufweist und durch eine Vakuumpumpe relativ zur Umgebung evakuiert werden kann.

18. Vorrichtung nach Anspruch 16 oder 17, **dadurch gekennzeichnet, dass** im Innenraum und der Umgebung der Kapselabfüllmaschine Druckmessgeräte angeordnet sind, mittels derer durch Druckdifferenzmessung der Unterdruck bestimmt wird.

19. Vorrichtung nach Anspruch 16, **dadurch gekennzeichnet, dass** im Innenraum der Kapselabfüllmaschine eine Düse zum Einstrahlen erwärmter Gase in die Kapselteile angeordnet ist.

20. Vorrichtung nach Anspruch 19, **dadurch gekennzeichnet, dass** der Düsenstrahl eine Breite von 5 cm - 50 cm, insbesondere 10 cm bis 30 cm aufweist.

21. Vorrichtung nach einem der vorangegangen Ansprüche, **dadurch gekennzeichnet, dass** die Kapselhalterung Mittel zum Kühlen aufweist.

22. Vorrichtung nach einem der vorangegangen Ansprüche, **dadurch gekennzeichnet, dass** die Mittel zum Kühlen eine Wasserkühlung, oder ein Kaltluftstrahl oder ein Peltier-Element sind.

23. Vorrichtung nach einem der Ansprüche 16 bis 20, **dadurch gekennzeichnet, dass** die Vorrichtung eine Steuereinrichtung mit einem Datenspeicher aufweist, in welchem die Druck- und /oder Temperaturdaten des Prozessgases abgelegt werden und wobei die Steuereinrichtung in Abhängigkeit vom Kapselmaterial und vom verwendeten Arzneimittel den Druck und/oder die Temperatur des Prozessgases steuert.

24. Software zum Steuern eines Verfahrens nach den Ansprüchen 1 bis 15 oder einer Vorrichtung nach den Ansprüchen 16 bis 23 **dadurch gekennzeichnet, dass** die Software die Verarbeitungsschritte und Steuerschritte
• Auslesen der Prozessparameter zur Dosierung des Arzneimittels in die Kapsel körper
• Erfassen der Messdaten von Druck und/oder Temperatur des Prozessgases
• Berechnung der Solldaten für Druck und/oder Temperatur des Prozessgases
• Regelung des Druckes und/oder der Temperatur des Prozessgases umfasst.
